Europäisches Patentamt

European Patent Office

Office européen des brevets

(11) Publication number: **0 186 522**
**B1**

(12) **EUROPEAN PATENT SPECIFICATION**

(45) Date of publication of patent specification: **14.03.90**

(21) Application number: **85309520.6**

(22) Date of filing: **30.12.85**

(51) Int. Cl.⁵: **C 12 N 15/00, C 07 H 21/04,**
**G 01 N 33/574, C 12 P 19/34,**
**G 01 N 33/58**

(54) **Cancer-specific DNA.**

(30) Priority: **27.12.84 JP 281206/84**

(43) Date of publication of application:
**02.07.86 Bulletin 86/27**

(45) Publication of the grant of the patent:
**14.03.90 Bulletin 90/11**

(84) Designated Contracting States:
**AT BE CH DE FR GB IT LI LU NL SE**

(56) References cited:
**NUCLEIC ACIDS RESEARCH, vol. 9, no. 9, 11**
**May 1981, London (GB); H.LAND et al.: "5'-**
**terminal sequences of eucaryotic mRNA can be**
**cloned with high efficiency", pp. 2251-2266**

(73) Proprietor: **SANKYO COMPANY LIMITED**
**No. 1-6, 3-chome Nihonbashi Honcho Chuo-ku**
**Tokyo (JP)**

(72) Inventor: **Endo, Hideya**
**6-22-33, Kasumigaoka Higashi-ku**
**Fukuoka-city Fukuoka-prefecture (JP)**

(74) Representative: **Ruffles, Graham Keith et al**
**MARKS & CLERK 57-60 Lincoln's Inn Fields**
**London WC2A 3LS (GB)**

# EP 0 186 522 B1

**Description**

This invention relates to nucleic acid sequences, and in particular it provides DNA which has a specific role in cancer cells.

In recent years, marked progress has been made in research on the mechanism of carcinogenesis. It has become clear that generation of a cancer can arise from activation of a proto-oncogene existing on the chromosome of a normal cell. However, it is not clear how cells are transformed by the activated proto-oncogene, and in particular by the protein product of the oncogene (cancer gene).

An object of this invention is to allow investigation and clarification of control abnormalities previously considered as causes for a diversity of changes in expressed traits which occur simultaneously with transformation of cells. A further object is the isolation of a gene which is expressed only in cancer cells, and is not expressed in normal cells. A related object is the provision of probes and assays for cancer, based on cancer-specific nucleic acid sequences. In this respect, by "cancer-specific" nucleic acid sequence is meant a nucleic acid sequence which has a specific role in cancerous cells, typically through expression in cancer cells.

The present invention provides a process for producing a cancer-specific DBA as defined below, which comprises the steps of:

(a) obtaining DNA from a cancer cell;
(b) synthesizing single-stranded DNA complementary to the RNA;
(c) producing double-stranded DNA from the single stranded cDNA;
(d) inserting the double-stranded DNA into a vector to give a recombinant DNA plasmid;
(e) transforming a host with the recombinant DNA plasmid;
(f) screening the transformed host for cancer-specific DNA; and
(g) isolating the desired, cancer-specific DNA.

By this process, the invention is able to provide cancer-specific DNA sequences. Such sequences will be of immense value, for example in the diagnosis of cancer through the use of labelled forms of the cancer-specific DNA. By way of illustration, such diagnosis may be performed using conventional hybridization techniques.

The DNA of the present invention may be single or double stranded. For example, the invention further provides complementary cancer-specific single-stranded DNA sequences, the single strands being complementary in the sense that they hybridize at least partially to give a cancer-specific double-stranded DNA sequence of this invention. The double-stranded DBA is itself suited for use in further research, such as through incorporation in a recombinant plasmid.

The present invention also embraces the mRNA coded by the double-stranded DNA, and labelled forms thereof which are again suited for use as probes.

It will be recognised that the genetic code is redundant, and nucleic acid bases can often be replaced by other bases without change in the function of the sequence. Accordingly, the present invention extends to DNA sequences which are functionally substantially homologous with the single or double-stranded DNA sequences of this invention.

In a preferred form, the present invention provides a DNA sequence corresponding to a DNA sequence specifically associated with cancerous cells and comprising:

(i) the base sequence (I):

2

```
5'  A T A T C T A A G T C A A T A T T A G G
    C A T G G A A A T C T T G G G G G C C T
    T C G A A T G T T A T C T C A G G C A T
    C T T G A A C T T G G G G C C C T T C A
    G T T T T G C A T C T G G G T C C T G A
    C A C A T C A A G G T T C A G C C T T G
    G G T G G G T T C A C A T C C A C C T C
    A G G G C C C T C T G C T T T G A A G C
    C A A G C A T A C T G A A C T T G G G C
    A T C T T C A T C T T G G G C A T C T T
    C A G G T G C C A G T C T G G G C C C T
    G C A C A T T C A C A T T C G G A A C A
    T C A A T G T T C A C T T T G G G G C C
    T T T G A T G T C A A C T T C G G A A C
    T T T A A T C T C A C C T T C C A C T T
    T G G G A A G A G A C A C G T C C A C A
    T C A C C C T T C A C T T T G G G G C C
    T T T C A G A T T C A G A T C A A A G T
    C A G G C A T G G A G A T C T T T G G G
    G G C C T T G A T G T T C A T C T C A G
    G C A T C T T G A A C T T G G G G C C T
    T T T A G T T T T G C A T C C G G A C C
    T C C A A T A T T C A C A T C C G G A A
    C A T C A A T G T C C A C-C C T G G G T
    C C T G A C A C A T C A A G G T C A G C
    C T T G G G C A G G C T C A T A T C C A
    C C T C T G G G C C C T C T G C T T T G
    A A G C C A G G C A T A C T G A A C T T
    G G G C A T C T T C A T C T T G G G C A
    T C T C C A G G T G C C A G T C T G G G
    C C C T G C A C A T C C A C A T C T G G
    A G C A T T G A T G A C C A C T T T G G
    G G C C T T T G A T G T C A A C T T C A      3';
```

or an allelic variant or mutant thereof; or

(ii) a base sequence adapted to be usable to a probe for the base sequence defined at (i).

Double-stranded DNA can readily be constructed comprising as first strand, the DNA sequence (I) or a substantially homologeous sequence, and as second strand, a substantially complementary DNA sequence. Such double-stranded DNA, especially a cDNA, can then be used for further investigations in to cancer, for example by inserting the DNA in to a plasmid, especially to give a recombinant plasmid including an expression promoter.

The RNA sequence coded by the double-stranded DNA is then also of interest for further research.

DNA containing the base sequences of the present invention can be expressed specifically in a wide spectrum of cancer cells without restriction to a particular species, but typically such DNA is not expressed in normal cells. Therefore, the DNA is useful for diagnosis of cancer, utilising this specificity, or the cancer thereby by use of an anti-messenger RNA to inhibit translation of the mRNA.

Labelled gene probes can readily be provided which comprise a labelled form of a DNA or RNA sequence of this invention, with the label typically being a radiolabel. The probes can then be used in a cancer assay provided by the present invention.

The assay of this invention typically involves testing for cancer by incubating a sample of RNA or denatured DNA incubated with a labelled gene probe, and examining the incubated system for hybridization.

In a preferred preparation process of this invention, DNA containing the base sequence (I) or a base sequence with the same functionality can be produced for example by the steps (a) to (h) shown below:

(a) isolating mRNA from cancer cells;

(b) synthesizing a single stranded cDNA with the use of this mRNA and a reverse transcriptase;

(c) synthesizing a double stranded DNA on the basis of this cDNA;

(d) ligating the double stranded DNA obtained into a plasmid vector;

(e) introducing the recombinant plasmid into a host, for example, *E. Coli* to transform said host;

(f) screening the transformed host with a cancer cell probe and with a normal cell probe to isolate a transformed host containing a cancer specific cDNA;

(g) isolating the plasmid containing the desired cDNA from transformed cells obtained by culturing of said host; and

(h) isolating the desired cloned DNA from the plasmid.

The mRNA should be isolated as promptly as possible after the cancer tissue has been obtained. If this approach is not possible, the tissue should desirably be frozen with liquid nitrogen and stored at $-80°C$.

In extracting the RNA from the cancer tissue, the guanidine thiocyanate-hot phenol method, the guanidine thiocyanate guanidine hydrochloride method, the guanidine thiocyanate-cesium chloride method, among others, may be employed. It is preferred to use the guanidine thiocyanate-cesium chloride method.

Since most of the mRNA existing in the cytoplasm of eucaryotic cells is known to have a poly(A) sequence at the 3'-end thereof, the mRNA can be purified by absorbing it on to an oligo(dT)cellulose column, followed by elution.

By way of the mRNA as the template, a complementary double stranded DNA can be synthesized with the use of a reverse transcriptase. Synthetic procedures include:

the $S_1$ nuclease method [Efstradiadis *et al*; Cell, 7, 279 (1976)];

the Land method (Land *et al*; Nucleic Acids Res. 9, 2251 (1981)];

the Okayama-Berg method (Okayama and Berg; Mol Cell Biol, 2, 161 (1982)]) and

the O Joon Yoo method (Joon Yoo *et al*; Prod Natl Acad Sci USA, 79, 1049 (1982)].

For the present invention, the Land method is preferred.

The resultant cDNA can be tailed with a homopolymer of one DNA (dA, dT, dC or dG) and inserted in to a suitably cleaved and tailed plasmid. For example, the cDNA can be inserted at the restriction endonuclease Pst I cleavage site of the plasmid pBR 322. The use of poly(dG) and poly(dC) is preferred for superior stability.

A host such as *E. coli* can then be transformed by the recombinant plasmid obtained, and the transformants selected for phenotypic traits unique to the transformed host. In the presently preferred process, the cDNA is inserted at the ampicillin resistance gene of pBR 322, and thus selection is on the basis of ampicillin sensitivity and tetracycline resistance.

From among the resultant clones, any clones are selected which contain cDNA corresponding to mRNA expressed specifically in cancers. To this end, it is preferred to utilize the colony hybridization method based on a probe labeled with $^{32}P$ [Grunstein and Hogness; Proc Natl Acad Sci USA, 72, 3961 (1975)].

The base sequence contained in the selected clones can then be determined, if desired. The Maxam-Gilbert method [Maxam and Gilbert; Proc Natl Acad Sci USA, 74, 560 (1977)] is preferred.

In a particularly preferred embodiment of this invention, the following steps are used to obtain and test the cancer-specific DNA:

(1) From rat ascites hepatoma cells such as the known cell lines AH60C, AH66, AH66F, AH109A, AH130 or AH414, mRNA is isolated and a cDNA clone library prepared. The cDNA close library is screened according to the differential colony hydridization method with the use of two probes, a single-stranded labeled cDNA prepared from mRNA of AH60C and one from normal liver. Any close which reacts only with the AH60C probe and does not react with the normal liver probe is selected as the candidate for the cancer-specific cDNA clone.

(2) Next, the cDNA clone selected is further screened for instance by the Northern blotting method to confirm the cancer-specific nature of the cDNA clone.

(3) Then, with the use of probe of the cDNA clone thus identified, reactivity with mRNA prepared from other tissues, such as cancers of rats, mice and humans and various normal tissues, can be examined according to the dot blotting method. The cancer-specificity can be expressed semi-quantitatively.

The present invention will now be illustrated by way of non-limiting example with reference to the accompanying drawings, in which:

Figure 1 is a Northern blot analysis of poly(A) RNA from normal liver and various cancer cells, when hybridizing with plasmid pAH1005.

Figure 2 is a semi-quantitative Northern dot blot assay of poly(A) RNA from normal liver and various cancers with the plasmid pAH1005.

## Example 1
### Preparation of cancer-specific cDNA

Separation of poly(A) RNA

Cell line AH60C represents readily available cells of a rat cancer induced by a p-dimethylaminoazobenzene. The cell line was grown as an ascites hepatoma. Using a Waring (Trade Mark) blender, the cultured AH60C cells were homogenized in a 5-fold volume excess of an aqueous solution of 6M guanidine isothiocyanate, 5 mM sodium citrate (pH 7.0), 0.1 M mercaptoethanol and 0.5% sarcosine.

The homogenate was further processed in 2.5 ml aliquots. 1 g of cesium chloride was added to each aliquot, and the mixture was overlaid on a solution of 5.7 M cesium chloride and 100 mM disodium ethylenediaminetetraacetic acid, followed by centrifugation at 80,000 × g and 20°C for 20 hours.

The resultant precipitated RNA was washed once with ethanol and then subjected to column chromatography on oligo(dT)cellulose, in order to obtain RNA bearing a poly(A) sequence at the 3'-end. The column chromatography was repeated to purify the poly(A) RNA.

Preparation of cDNA library and screening

A library comprising about 1,500 cDNA clones and prepared from the poly(A) RNA of AH60C using the Land method. Insertional inactivation of the β-lactamase gene of the well known plasmid pBR 322 was used for selection of recombinant plasmid clones.

Thus, in known manner, for each member of the library, a homopolymer tail was attached to the 3'-ends of the double stranded cDNA. The cDNA was then allowed to form recombinant plasmids with Pat I-linearized pBR 322 which had been tailed at the 3'-ends with a complimentary homopolymer. The resultant plasmids were used to transform *E. coli,* and clones selected on the basis of resistance to tetracycline and sensitivity to ampicillin.

Colony hybridization by the method of Grunstein and Hogness was then employed to select cancer specific cDNA clones. For each recombinant plasmid clone, two sheets of nitrocellulose filter were inoculated with the cDNA clone. One filter was hybridized with $^{32}$P-labeled non-cancerous rat liver cDNA prepared by reverse transcription of Donryu for normal liver poly(A) RNA, and the other sheet was hybridized with a $^{32}$P-labeled cDNA probe of AH60C. The clones which reacted only with the AH60C probe and which did not react with the rat normal probe were selected as candidates for a cancer-specific cDNA clone. From among these candidates, a cancer-specific cDNA clone was identified by the Northern blotting method and designated pAH1005.

This cancer-specific cDNA clone pAH1005 was found to have a cDNA of about 700 bp inserted at the Pst I site of pBR 322. The base sequence of the DNA fragment and determined according to the Maxam-Gilbert method, and found to be the base sequence (I) given above.

## Example 2
### Testing of the cancer-specific cDNA

Cell sources

Readily available cancerous cells AH66, AH130, AH414, AH66F and Walker 250 were employed for testing the cDNA of this invention. Ascite hepatoma strains AH66, AH130, AH414 are transplantable rat cancers independently induced by p-dimethylaminoazobenzene AH66F is a strain derived from AH66. These cell strains embrace various phenotypes for instance with regard to survival time, transplantability, tumor island formation, metastasis and histological type, and were maintained by continuous intraperitoneal transplantation into male Donryu rats. Walker 256 carcinosarcoma was obtained from Wistar rats.

Normal rat liver, brain, lung, stomach, kidney and spleen tissue samples were obtained from cancer-free Donryu rats. Regenerated rat liver tissue was prepared by partial exciting of rat liver according to the method of Higgins and Anderson (Arch Pathol, *12,* 186—202) and removal of the liver after 12 hours. Fetal rat liver was obtained from rat embryos at 17 days gestation. Normal mouse liver was obtained from C3H/He mice.

Human primary hepatoma and associated peripheral liver tissue were surgically removed from a patient.

Cell lines were used for rat carcinoma strain AH109, mouse carcinoma strain NH134 (ascites hepatoma), Lewis lung cancer, Sarcoma 180, human carcinoma strain MK (from stomach cancer), and MX-1 (from breast cancer).

RNA-DNA hydridization

Gel electrophoresis was carried out on 1.5% agarose/6%(v/v) formaldehyde gel using 5 microgram samples per lane of polyA mRNA derived from (A) rat normal liver and AH60C (lanes 1 and 2 for the first experiment), and (B) AH66F, Walker 256 carcinosarcoma mouse normal liver, MH 134, normal human liver tissue adjacent a hepatoma, MX-1, and MK (lanes 1 to 7, respectively, for the second experiment). The RNA was then transferred to a nitrocellulose filter which was baked at 80°C for 2 hours. Pre-hybridization was performed, followed by washing with 0.3 M sodium chloride/0.03 M sodium citrate/0.1% sodium dodecyl sulphate. The prepared filter was hybridized with the $^{32}$P-labeled cDNA of pAH1005 according to the method of Thomas [Prod Natl Acad Sci, USA, 77, 5201—5205 (1980)].

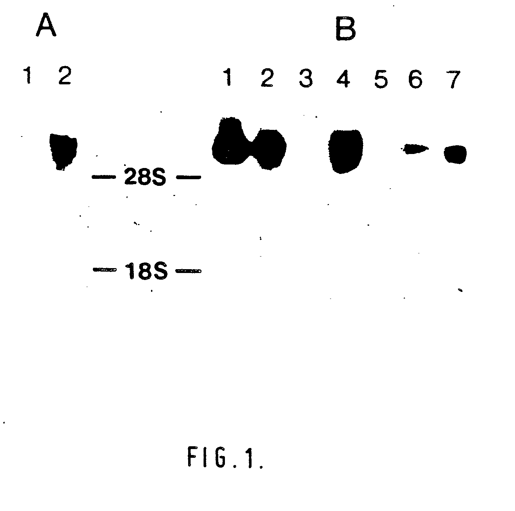

The results for the first and second experiments are shown in Figures 1A and 1B, respectively.

From Fig. 1A, a single strong band corresponding to a size to 6.8 Kb was found only in the blot of the mRNA of AH60C and not in the blot for rat normal liver.

Similarly, from Fig. 1B, the same 6.8Kb band was found variously for samples of a different rat ascites hepatoma strain AH66F, a solid cancer strain Walker 256 carcinosarcoma, a mouse tumor strain MH134 and two human tumor strains MK, MX-1. However, for mouse normal liver and human normal peripheral liver tissue adjacent a hepatoma, no such band can be seen.

Thus, the isolated cDNA clone was derived from mRNA of 6.8 Kb, and the mRNA is expressed in a wide variety of cancers which are not species-specific, but it is not expressed in normal liver.

Semi-quantitative RNA dot blot assay

The method of Thomas was used again, except that the nitrocellulose filter was treated with 3M sodium chloride/0.3M sodium citrate.

As the samples, in addition to AH60C, the three ascites hepatoma strains AH66, AH66F, AG414 and solid cancer strain Walker 256 carcinosarcoma were used as the source for supplying poly(A), RNA of rat cancer. Poly(A) RNA from normal adult liver, and fetal liver regenerated liver, brain, lung, stomach, kidney and spleen of rat were used as non-tumor controls.

Further, poly(A) RNA form human carcinoma strain MK and the primary liver cancer and its peripheral liver tissue excised from a patient were assayed.

Poly(A) RNA from normal and cancer tissue was diluted stepwise to 0.0313 microgram, starting from 1 microgram, and spotted on a nitrocellulose filter and dried in air. After baking at 80°C pre-hybridization was performed. The filter was then hybridized with the cloned DNA of pAH1005 labeled by the nick translation method [Rigby et al, J Mol Biol, 113, 237—251, 1977)].

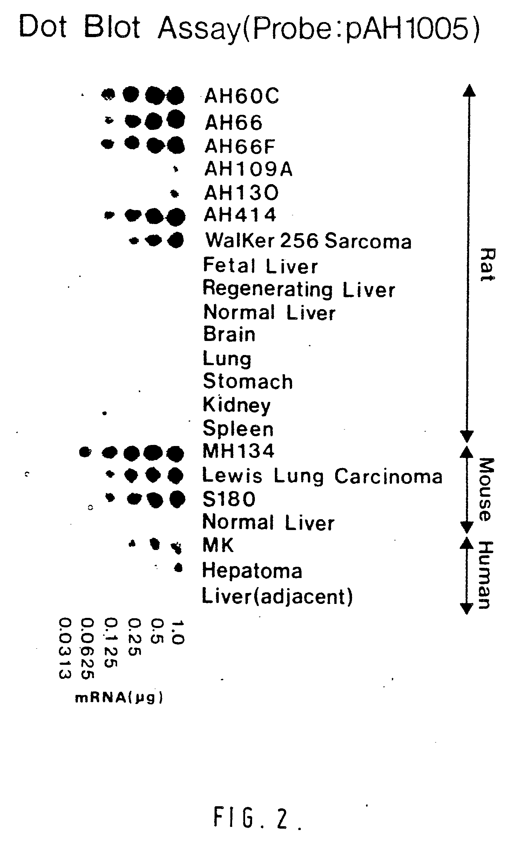

The results are shown in Figure 2.

As is apparent from Figure 2, RNA corresponding to the isolated clone was found expressed by all the cancer cells which were examined, but substantially not in any of the normal classes.

# EP 0 186 522 B1

**Claims**

1. A DNA sequence corresponding to a DNA sequence specifically associated with cancerous cells and comprising:
(i) the base sequence (1):

```
5'  A T A T C T A A G T C A A T A T T A G G
    C A T G G A A A T C T T G G G G G C C T
    T C G A A T G T T A T C T C A G G C A T
    C T T G A A C T T G G G G C C C T T C A
    G T T T T G C A T C T G G G T C C T G A
    C A C A T C A A G G T T C A G C C T T G
    G G T G G G T T C A C A T C C A C C T C
    A G G G C C C T C T G C T T T G A A G C
    C A A G C A T A C T G A A C T T G G G C
    A T C T T C A T C T T G G G C A T C T T
    C A G G T G C C A G T C T G G G C C C T
    G C A C A T T C A C A T T C G G A A C A
    T C A A T G T T C A C T T T G G G G C C
    T T T G A T G T C A A C T T C G G A A C
    T T T A A T C T C A C C T T C C A C T T
    T G G G A A G A G A C A C G T C C A C A
    T C A C C C T T C A C T T T G G G G C C
    T T T C A G A T T C A G A T C A A A G T
    C A G G C A T G G A G A T C T T T G G G
    G G C C T T G A T G T T C A T C T C A G
    G C A T C T T G A A C T T G G G G C C T
    T T T A G T T T T G C A T C C G G A C C
    T C C A A T A T T C A C A T C C G G A A
    C A T C A A T G T C C A C C C T G G G T
    C C T G A C A C A T C A A G G T C A G C
    C T T G G G C A G G C T C A T A T C C A
    C C T C T G G G C C C T C T G C T T T G
    A A G C C A G G C A T A C T G A A C T T
    G G G C A T C T T C A T C T T G G G C A
    T C T C C A G G T G C C A G T C T G G G
    C C C T G C A C A T C C A C A T C T G G
    A G C A T T G A T G A C C A C T T T G G
    G G C C T T T G A T G T C A A C T T C A      3';
```

7

or an allelic variant or mutant thereof; or

(ii) a base sequence adapted to be usable as a probe for the base sequence defined at (i).

2. A double-stranded DNA which comprises:

a first strand, comprising the DNA sequence defined at (i) in claim 1 and a second strand which is a substantially complementary DNA sequence and which is a DNA sequence as defined at (ii) in claim 1.

3. An RNA sequence coded by the double-stranded DNA of claim 2.

4. A recombinant plasmid including a double-stranded DNA according to claim 2.

5. A recombinant plasmid according to claim 4, further including an expression promoter for the said double-stranded DNA.

6. A labelled gene probe which comprises a labelled form of a DNA sequence according to claim 1.

7. A labelled gene probe which comprises a labelled form of an RNA sequence according to claim 3.

8. A labelled gene probe according to claim 6 or 7, wherein the label is a radiolabel.

9. A method of testing for cancer, wherein a sample of RNA or denatured DNA is incubated with a labelled gene probe according to claim 6, 7 or 8, and examined for hybridization.

10. A process for producing a cancer-specific DNA according to claim 1, which comprises the steps of:

(a) obtaining RNA from a cancer cell;

(b) synthesizing single-stranded cDNA complementary to the RNA;

(c) producing double-stranded DNA from the single stranded cDNA;

(d) inserting the double-stranded DNA into a vector to give a recombinant DNA;

(e) transforming a host with the recombinant DNA;

(f) screening the transformed host for cancer-specific DNA; and

(g) isolating the desired cancer-specific DNA from the cloned host.

**Patentansprüche**

1. Ein DNA-Sequenz, welche einen spezifisch mit Krebszellen assoziierten DNA-Sequenz entspricht und (i) die Basen-Sequenz (I):

```
5'   A T A T C T A A G T C A A T A T T A G G
     C A T G G A A A T C T T G G G G C C T
     T C G A A T G T T A T C T C A G G C A T
     C T T G A A C T T G G G G C C C T T C A
     G T T T T G C A T C T G G G T C C T G A
     C A C A T C A A G G T T C A G C C T T G
     G G T G G G T T C A C A T C C A C C T C
     A G G G C C C T C T G C T T T G A A G C
     C A A G C A T A C T G A A C T T G G G C
     A T C T T C A T C T T G G G C A T C T T
     C A G G T G C C A G T C T G G G C C C T
     G C A C A T T C A C A T T C G G A A C A
     T C A A T G T T C A C T T T G G G G C C
     T T T G A T G T C A A C T T C G G A A C
     T T T A A T C T C A C C T T C C A C T T
     T G G G A A G A G A C A C G T C C A C A
     T C A C C C T T C A C T T T G G G G C C
     T T T C A G A T T C A G A T C A A A G T
     C A G G C A T G G A G A T C T T T G G G
     G G C C T T G A T G T T C A T C T C A G
     G C A T C T T G A A C T T G G G G C C T
     T T T A G T T T T G C A T C C G G A C C
     T C C A A T A T T C A C A T C C G G A A
     C A T C A A T G T C C A C C C T G G G T
     C C T G A C A C A T C A A G G T C A G C
     C T T G G G C A G G C T C A T A T C C A
     C C T C T G G G C C C T C T G C T T T G
     A A G C C A G G C A T A C T G A A C T T
     G G G C A T C T T C A T C T T G G G C A
     T C T C C A G G T G C C A G T C T G G G
     C C C T G C A C A T C C A C A T C T G G
     A G C A T T G A T G A C C A C T T T G G
     G G C C T T T G A T G T C A A C T T C A    3';
```

oder ein von deren allelischen Varianten oder Mutanten; oder

(ii) eine Basen-Sequenz umfaßt, welche zur Verwendung ein Probe für die in (i) definierte Basen-Sequenz geeignet ist.

2. Eine Doppel-Strang-DNA, welche

eine die in Anspruch 1 unter (i) definierte DNA-Sequenz aufweisenden ersten Strang und

einen zweiten Strang umfaßt, welcher eine substantiell komplementäre DNA-Sequenz darstellt und welcher eine im Anspruch 1 unter (ii) definierte DNA-Sequenz ist.

3. Eine RNA-Sequenz, welche durch die Doppel-Strang-DNA gemäß Anspruch 2 codiert ist.

4. Ein rekombinantes Plasmid, welches eine Doppel-Strang-DNA gemäß Anspruch 2 enthält.

5. Ein rekombinantes Plasmid gemäß Anspruch 4, welches weiters einen Expressions-Promotor für die genannte Doppel-Strang-DNA aufweist.

6. Eine markierte Gen-Probe, welche eine markierte Form einer DNA-Sequenz gemäß Anspruch 1 aufweist.

7. Eine markierte Gen-Probe, welche eine markierte Form einer DNA-Sequenz gemäß Anspruch 3 aufweist.

8. Eine markierte Gen-Probe gemäß Anspruch 6 oder 7, worin die Markierung eine Radio-Markierung bzw. Markierung mittels Radio-Nuclid ist.

9. Eine Methode zur Krebs-Testung, wobei eine Probe einer RNA oder einer denaturierten DNA mit einer markierten Gen-Probe gemäß Anspruch 6, 7 oder 8 inkubiert und auf Hybridisierung geprüft wird.

10. Ein Verfahren zur Herstellung einer Krebsspezifischen DNA gemäß Anspruch 1, welches die folgenden Schritte umfaßt:

(a) Erhalt der RNA aus einer Krebs-Zelle;

(b) Synthetisierung einer zu der RNA komplementären Einzel-Strang-cDNA;

(c) Herstellung einer Doppel-Strang-DNA aus der Einzel-Strang-cDNA;

(d) Einfügen der Doppel-Strang-DNA in einen Vektor, um eine rekombinante DNA zu erhalten;

(e) Transformation eines Wirtes mit der rekombinanten DNA;

(f) Screenen des transformierten Wirtes auf krebsspezifische DNA; und

(g) Isolieren der gewünschten krebsspezifischen DNA aus dem geklonten Wirt.

**Revendications**

1. Séquence d'ADN correspondant à une séquence d'ADN spécifiquement associée aus cellules cancéreuses et comprenant:
(i) la sequenz de bases (I):

```
5'  A T A T C T A A G T C A A T A T T A G G

    C A T G G A A A T C T T G G G G G C C T

    T C G A A T G T T A T C T C A G G C A T

    C T T G A A C T T G G G G C C C T T C A

    G T T T T G C A T C T G G G T C C T G A

    C A C A T C A A G G T T C A G C C T T G

    G G T G G G T T C A C A T C C A C C T C

    A G G G C C C T C T G C T T T G A A G C

    C A A G C A T A C T G A A C T T G G G C

    A T C T T C A T C T T G G G C A T C T T

    C A G G T G C C A G T C T G G G C C C T

    G C A C A T T C A C A T T C G G A A C A

    T C A A T G T T C A C T T T G G G G C C

    T T T G A T G T C A A C T T C G G A A C

    T T T A A T C T C A C C T T C C A C T T

    T G G G A A G A G A C A C G T C C A C A

    T C A C C C T T C A C T T T G G G G C C

    T T T C A G A T T C A G A T C A A A G T

    C A G G C A T G G A G A T C T T T G G G

    G G C C T T G A T G T T C A T C T C A G

    G C A T C T T G A A C T T G G G G C C T

    T T T A G T T T T G C A T C C G G A C C

    T C C A A T A T T C A C A T C C G G A A

    C A T C A A T G T C C A C C C T G G G T

    C C T G A C A C A T C A A G G T C A G C

    C T T G G G C A G G C T C A T A T C C A

    C C T C T G G G C C C T C T G C T T T G

    A A G C C A G G C A T A C T G A A C T T

    G G G C A T C T T C A T C T T G G G C A

    T C T C C A G G T G C C A G T C T G G G

    C C C T G C A C A T C C A C A T C T G G

    A G C A T T G A T G A C C A C T T T G G

    G G C C T T T G A T G T C A A C T T C A     3';
```

ou une variante allélique ou un mutant de celle-ci; ou

(ii) une sequence de bases adaptée pour être utilisable comme sonde pour la séquence de bases définie au (i).

2. ADN bicaténaire caractérisé en ce qu'il comprend:

un premier brin comprenant la séquence d'ADN definie au (i) dans la revendication 1 et un second brin qui est une séquence d'ADN pratiquement complémentaire et qui est une séquence d'ADN comme définie au (ii) dans la revendication 1.

3. Séquence d'ADN codée par l'ADN bicaténaire de la revendication 2.

4. Plasmide recombinant caractérisé en ce qu'il inclut un ADN bicaténaire selon la revendication 2.

5. Plasmide recombinant selon la revendication 4, caractérisé en ce qu'il inclut de plus un promoteur pour l'expression dudit ADN bicaténaire.

6. Sonde de géne marquée, caractérisée en ce qu'elle comprend une forme marquée d'une séquence d'ADN selon la revendication 1.

7. Sonde de géne marquée, caractérisée en ce qu'elle comprend une forme marquée d'une séquence d'ARN selon la revendication 3.

8. Sonde de gène marquée selon la revendication 6 où 7, caractérisée en ce que la marque est une marque radioactive.

9. Procédé de détection du cancer, caractérisé en ce qu'un échantillon d'ARN ou d'ADN dénaturé est incubé avec une sonde de gène marquée selon la revendication 6, 7 ou 8, et examiné pour déceler l'hybridation.

10. Procédé pour produire un ADN spécifique du cancer selon la revendication 1, caractérisé en ce qu'il comprend les étapes consistant à:

a) obtenir l'ARN à partir d'une cellule cancéreuse;

b) synthétiser l'ADNc monocaténaire complémentaire de l'ARN;

c) produire l'ADN bicaténaire à partir ou l'ADNc monocaténaire;

d) insérer l'ADN bicaténaire dans un vecteur pour donner un ADN recombinant;

e) transformer un hôte avec l'ADN recombinant;

f) cribler l'hôte transformé pour déceler l'ADN spécifique dy cancer; et

g) isoler l'ADN specifique dy cancer, désiré à partir de l'hôte cloné.

12

A B

1 2 1 2 3 4 5 6 7

— 28S —

— 18S —

FIG.1.

# Dot Blot Assay(Probe:pAH1005)

AH60C
AH66
AH66F
AH109A
AH13O
AH414
WalKer 256 Sarcoma
Fetal Liver
Regenerating Liver
Normal Liver
Brain
Lung
Stomach
Kidney
Spleen
MH134
Lewis Lung Carcinoma
S180
Normal Liver
MK
Hepatoma
Liver(adjacent)

Rat

Mouse

Human

1.0
0.5
0.25
0.125
0.0625
0.0313
mRNA(μg)

FIG. 2.